# EUROPEAN PATENT SPECIFICATION

(11) **EP 4 267 115 B1**
(45) Date of publication and mention of the grant of the patent: **28.01.2026**
(21) Application number: 21843911.5
(22) Date of filing: 20.12.2021
(51) Int. Cl.: A61K 31/00, A61K 47/69, A61K 47/54

(54) **NANOSTRUCTURED DELIVERY SYSTEM FOR TUMOR TREATMENT**
NANOSTRUKTURIERTES ABGABESYSTEM ZUR TUMORBEHANDLUNG
SYSTÈME D'ADMINISTRATION NANOSTRUCTURÉ POUR LE TRAITEMENT DE TUMEURS

(30) Priority: 23.12.2020 EP 20217124
(43) Date of publication of application: 01.11.2023
(73) Proprietor: SmartDyeLivery GmbH, 07743 Jena (DE)
(72) Inventor: HOCHHAUS, Andreas, 99423 Weimar (DE); ERNST, Philipp, 07743 Jena (DE); ENZENSPERGER, Christoph, 07745 Jena (DE); BAUER, Michael, 07743 Jena (DE)
(74) Representative: Kehl, Ascherl, Liebhoff & Ettmayr Patentanwälte Partnerschaft mbB
(86) International application number: PCT/EP2021/086860
(87) International publication number: WO 2022/136303

(56) References cited:
- EP-A1- 2 848 262
- WO-A1-2011/116142
- ADRIAN T. PRESS ET AL: "Cell type-specific delivery of short interfering RNAs by dye-functionalised theranostic nanoparticles", NATURE COMMUNICATIONS, vol. 5, no. 1, 1 December 2014 (2014-12-01), XP055557357, DOI: 10.1038/ncomms6565
- PRESS ADRIAN T ET AL: "Targeted delivery of a phosphoinositide 3-kinase [gamma] inhibitor to restore organ function in sepsis", EMBO MOLECULAR MEDICINE, vol. 13, no. 10, 2 September 2021 (2021-09-02), US, XP093184549, ISSN: 1757-4676, Retrieved from the Internet <URL:https://onlinelibrary.wiley.com/doi/full-xml/10.15252/emmm.202114436> DOI: 10.15252/emmm.202114436
- MCMAHON HARVEY T. ET AL: "Molecular mechanism and physiological functions of clathrin-mediated endocytosis", NATURE REVIEWS MOLECULAR CELL BIOLOGY, vol. 12, no. 8, 22 July 2011 (2011-07-22), London, pages 517 - 533, XP093184551, ISSN: 1471-0072, Retrieved from the Internet <URL:http://www.nature.com/articles/nrm3151> DOI: 10.1038/nrm3151
- KOTANI NAOKI ET AL: "Expression and Transport Function of Drug Uptake Transporters in Differentiated HepaRG Cells", MOLECULAR PHARMACEUTICS, vol. 9, no. 12, 6 November 2012 (2012-11-06), US, pages 3434 - 3441, XP093184368, ISSN: 1543-8384, DOI: 10.1021/mp300171p
- VAN CALSTER BEN ET AL: "Sensitivity and Specificity Can Change in Opposite Directions When New Predictive Markers Are Added to Risk Models", MEDICAL DECISION MAKING, vol. 34, no. 4, 30 December 2013 (2013-12-30), pages 513 - 522, XP093185137, ISSN: 0272-989X, Retrieved from the Internet <URL:https://journals.sagepub.com/doi/full/10.1177/0272989X13513654> DOI: 10.1177/0272989X13513654
- X. YANG ET AL: "Near IR Heptamethine Cyanine Dye-Mediated Cancer Imaging", CLINICAL CANCER RESEARCH, vol. 16, no. 10, 21 April 2010 (2010-04-21), pages 2833 - 2844, XP055149538, ISSN: 1078-0432, DOI: 10.1158/1078-0432.CCR-10-0059
- YUAN JIANLIN ET AL: "Near-infrared fluorescence imaging of prostate cancer using heptamethine carbocyanine dyes", MOLECULAR MEDICINE REPORTS, vol. 11, no. 2, 29 October 2014 (2014-10-29), GR, pages 821 - 828, XP055808245, ISSN: 1791-2997, DOI: 10.3892/mmr.2014.2815

## Description

### Field of the invention

The present invention relates to a nanostructured delivery system as defined in claim 1. The invention relates further to a pharmaceutical composition as defined in 6, to a method for targeted transport as defined in claim 8 and to a use of the nanostructured delivery system or of the pharmaceutical composition of the invention as defined in claim 10.

### Background

There is increasing recognition of the importance of organ-specific drug delivery as a way for optimizing drug efficacy. In the case of cancer therapy, the ability to target a specific organ or tissue allows for enhanced efficacy and fewer non-specific side-effects of anti-cancer drugs. Moreover, with targeted drug delivery, inter-subject variation in the expression and activity of drug disposition pathways manifesting as marked variation of the pharmacokinetic profile of such drugs may be circumvented. The use of nanoparticles that transport active ingredients into a certain tissue in a targeted manner is known in the prior art (C. Sheridan, Proof of concept for next-generation nanoparticle drugs in humans. Nat Biotechnol, 2012 30(6): pp. 471-3; S. E. Gratton et al., The effect of particle design on cellular internalization pathways. Proc Natl Acad Sci USA, 2008 105 (33): pp. 11613-8). These nanoparticles are used in tumor therapy and function according to the following mechanisms: the nanoparticle is provided with either a shell layer or an antibody. If coated with an aqueous shell layer, the nanoparticle is rendered unidentifiable for the immune system. If such nanoparticle is injected and not attacked by the immune system, it diffuses through the fenestrated blood vessels, which have significantly larger orifices (fenestrations) in tumor tissue compared with normal blood vessels. The nanoparticle is then absorbed by the surrounding cells which also are characterized by an increased permeability in comparison with healthy cells. The processes of passive enrichment of nanoparticles, liposomes or macromolecules as described above is referred to as EPR effect ("enhanced permeability and retention") and thus refers to passive drug targeting. Accordingly, the disadvantage lies in the fact that not only tumor cells take up the nanoparticle but also healthy cells - to which the nanoparticle is transported nonspecifically via the blood vessels. This can lead to serious adverse effects. If the nanoparticle is decorated with antibodies on its surface after being synthesized, cells are targeted which are expressing surface-antigens to which these antibodies bind. This transport mechanism is also passive and nonselective.

An adenocarcinoma is an neoplasm of epithelial tissue of glandular origin and/or having glandular characteristics. Several of the most common forms of cancer are adenocarcinomas, which are localized at common sites such as the breast, lung, prostate, and the gastrointestinal tract (colon, rectum, pancreas, stomach, esophagus). Adenocarcinomas may be diagnosed histologically using light microscopy, wherein the diagnosis is usually based on the identification of glandular structures. In poorly differentiated adenocarcinomas, immunohistochemistry (IHC) is an important for diagnosing the type of adenocarcinoma, in cases where only minimal glandular formation or no glandular formation is observed under light microscopy. Diagnostic IHC (see, e.g., Dabbs (ed): Diagnostic Immunohistochemistry) is a well-known tool in the differential diagnosis of adenocarcinomas: e.g., antibodies against cytokeratins may be used for identification of carcinomas such as cytokeratin 7 (CK-7) when diagnosing adenocarcinoma of the lung. These adenocarcinomas often exhibit mutations in the epidermal growth factor receptor (EGFR), anaplastic lymphoma kinase (ALK) and c-ROS oncogene 1(ROS-1). PD-L1 (programmed cell death 1 ligand 1) IHC assays are performed in advanced lung cancers. For example, positive IHC staining for CK-7, GATA-binding protein 3 (GATA-3), and gross cystic fluid protein 15 is suggestive of breast adenocarcinoma. For example, positive IHC staining for prostate-specific antigen (PSA) is specific for adenocarcinoma of the prostate, while nuclear basal protein P63 is used for differentiating normal prostatic tissue from adenocarcinoma of the prostate. for example, positive IHC staining of caudal type homeobox 2 (CDX-2) or cytokeratin-20 (CK-20) in conjunction with a negative CK-7 staining is suggestive of colorectal adenocarcinoma. For example, positive IHC staining for CK-7, Paired-Box-Protein 8 (PAX 8), and Wilms' tumor protein 1 (WT-1) is suggestive of ovarian adenocarcinoma. For example, positive IHC staining for thyroglobulin and thyroid transcription factor-1 (TTF-1) is suggestive of adenocarcinoma of thyroid.

There is extensive evidence showing that the metabolism of both exogenous and endogenous substances influences the growth characteristic of many cancer tissues. These metabolic changes may be categorized depending on the metabolic change involved, i.e. changes in the glucose-, amino acid-, and lipid-processing pathways. Nutrient transporters, such as glucose and amino acid transporters, often are differentially regulated in cancer cells on a transcriptional and post-translational level, supporting high proliferation levels and pathologic growth of the cells (Zhang, et al., 2018 The SLCO genes (solute carriers of the organic anion transporting subfamily) encode organic anion transporting polypeptides (OATPs), which have been implicated recently to play a role in cancer development as well as in cancer therapy (Obaidat et al., 2012). In particular, it was found that the expression of OATPs may be altered in different types of cancer. In consequence, it is hypothesized that OATP expression may play a potential role in the development and progression of cancer as well as the disposition of anticancer drugs.

Nanoparticles transporting active ingredients our known from the prior art and fall into different classes: simple delivery systems such as micelles, nano-emulsions, nanoliposomes, solid lipid microparticles, polymer and hydrogel particles, and complex systems which are fabricated by applying structural design principles to modify the properties and functionalities of simple delivery systems. Such complex nanoparticles are, for example, liposomes carrying a protective hydrophilic layer consisting of flexible hydrophilic polymers such as polyethylene glycol (PEG). By providing such coating, the clearance of the complex nanoparticle from the reticuloendothelial system (RES) is diminished such that these nanoparticles have a prolonged circulation time and an improved pharmacokinetic profile. Another example for a complex nanoparticle are poly(lactic-co-glycolic acid (PLGA)-based polymeric nanoparticles which provide, compared with other nanocarriers such as liposomes and micelles, the advantages of improved stability, high loading capacity, sustained drug release, non-immunogenic property, reduced drug toxicity, improved bioavailability, biocompatibility, biodegradability , enhanced therapeutic efficacy of an entrapped drug and versatile surface modification. PLGA based nanoparticles may be surface-modified using surfactants and/or other surface modifications conferring desired surface characteristics (i.e., zeta potential and hydrophilicity) for improved uptake e.g. into solid tissues or tumors via adsorptive transcytosis. Press et al. demonstrated that PLGA nanoparticles functionalized with a polymethine dye possess the ability to be taken up selectively by liver and kidney via clathrin-mediated endocytosis due to their affinity for transmembrane carrier proteins.

EP 2 848 262 A1 describes nanostructured delivery systems comprising polymethine dyes, and WO 2011/116142 A1; Yang, X, et al., "Near IR Heptamethine Cyanine Dye-mediated Cancer Imaging", Clinical Cancer Research, Vol 16, pages 2833-2844, (2010); and Yuan, J, et al, "Near-infrared fluorescence imaging of prostate cancer using heptamethine carbocyanine dyes", Molecular Medicine Reports, Vol. 11, pages 821-828 (2014) describe that polymethine dyes may be taken up by the disclosed adenocarcinomas. Kotani, N., et al., "Expression and transport function of drug uptake transporters in differentiated HepaRG cells", Molecular Pharmaceutics, Vol. 9, pages 3434-3441 (2012) investigates OATP1B1-mediated drug-drug interactions.

There is a need for an active and selective transport and/or delivery system in the context of tumor therapy, wherein the active transport takes place selectively into tumor tissue by means of a specific targeting unit, such that the pharmaceutically active ingredient may be transported into the tissue at the same time. The present invention provides for such as selective transport and/or delivery system into adenocarcinomatous cancer tissues characterized by an altered expression of one or more *SLCO* genes.

### Description of the invention

In a first aspect, the invention relates to a nanostructured delivery system as defined in claim 1.

As used in the invention, the "nanostructured delivery system" refers to a system comprising at least one or more polymer and/or lipid; if it comprises one or more polymer, it is referred to herein as "nanoparticle"; if it comprises one or more lipid it is referred to herein as a "liposome." If the nanostructured delivery system according to the invention comprises both polymers and lipids, it is referred to herein as "nanoparticle" or as "liposome." Accordingly, the terms "nanoparticle" and "liposome" are used synonymously according to the invention and also relate to a nanostructured delivery system comprising both polymers and lipids. Nanoparticles are structures which are smaller than 1 µm in size and may be constructed of a plurality of molecules. In general, they are characterized by a high surface-area-to-volume ratio and thus offer great chemical reactivity. These nanoparticles may consist of polymers wherein these polymers are characterized by the fact that certain units (monomers) are repeating units. The polymers are covalently bonded to one another by the chemical reaction of these monomers (polymerization). If some of these polymers have hydrophobic properties, they may form nanoscale structures (e.g., nanoparticles, micelles, vesicles) in an aqueous environment. Due to their hydrophobic properties, lipids may also be used to form nanoparticles (micelles, liposomes).

According to the invention, the term "adenocarcinoma" denotes is a neoplasm of epithelial tissue of glandular origin and/or having glandular characteristics Herein, the subject suffering from an adenocarcinoma is a mammal, preferably a human.

According to the invention, the term "gene expression" refers to the process by which information from a gene is used in the synthesis of a functional gene product, wherein in the gene product may be a peptide, a protein or any kind of RNA. In the context of the present invention, gene products resulting from the process of gene expression are functional proteins. The term "gene expression alteration" refers to an alteration in protein synthesis of the respective gene, which is present in a neoplastic cell of a tissue but not, or not to the same extent, in a normal, non-neoplastic cell of said tissue. An example for such a gene expression alteration is the up- or down-regulation of a protein in a neoplastic tissue, wherein the expression level is either increased or decreased compared to a normal tissue. A further example of an altered gene expression relates to a differential cellular distribution of a protein in a neoplastic cell, for example a shift from a regular localization at the cell-membrane to an intracellular compartment in the neoplastic cell. A further example relates to an alteration affecting gene function resulting in a protein with enhanced (gain of) or diminished (loss of) function.

As mentioned above, the *SLCO* genes (solute carriers of the organic anion transporting subfamily) encode organic anion transporting polypeptides (OATP). To date, there are 11 known human OATPs, divided into six families based on amino acid sequence identity *(SLCO1A2, SLCO1B1, SLCO1B3, SLCO1C1; SLCO2A1, SLCO2B1; SLCO3A1; SLCO4A1, SLCO4C1; SLCO5A1;* and *SLCO6A1*). All OATPs have 12 transmembrane domains with their respective amino and carboxy termini facing the intracellular space. OATPs mediate transport of their substrates via an electroneutral process wherein the influx of extracellular OATP substrates is accompanied by the efflux of intracellular anions such as bicarbonate.

As mentioned above, the use of nanoparticles that transport active ingredients into a certain tissue in a targeted manner ("targeted transport") is known from the prior art. "Targeted transport" (or cell-specific transport) is understood to refer to the targeted and selective accumulation of the vehicle at a desired site of action; in practice, targeted transport is used to transport an active ingredient to a specific tissue/cell population ("targeted drug delivery"), such that the active ingredient is released at a desired site of action, thereby increasing the efficacy of action of the active ingredient and reducing its systemic side effects. Active ingredients that are transported often include antibodies, peptides or small molecules, such as oligonucleotides or nucleic acids.

The at least one polymethine dye comprised in the nanostructured delivery system mediates the targeted transport of the nanostructured delivery system into said adenocarcinoma cells via the expression product of the one or more *SLCO1* genes. The adenocarcinoma comprises adenocarcinoma cells in which said gene expression alteration affects *SLCO1* genes; it is most preferred, when the adenocarcinoma comprises adenocarcinoma cells in which said gene expression alteration affects the *SCLO1B3* gene. Of all OATPs, the respective roles of OATP1A2, OATP1B1, OATP1B3 and OATP2B1 in normal tissues have been characterized most extensively, in particular their role in the uptake of pharmacological substances. In these tissues, OATP1 B1 and OATP1B3 are expressed in the basal lateral membrane of sinusoidal hepatocytes with a perivenous-to-periportal expression gradient, while OATP1A2 is predominantly expressed at the blood-brain barrier and in duodenal enterocytes.

The adenocarcinoma to be treated by the using the nanostructured delivery system of the invention is a cancer selected from the group comprising gastrointestinal cancer, lung cancer, prostate cancer, breast cancer. As used in the invention, the term "gastrointestinal cancer" refers to a cancer originating from neoplastic cells of the gastrointestinal tract, for instance gastric cancer, colon cancer, colorectal cancer, rectal cancer, pancreatic cancer, gallbladder cancer, liver cancer.

In a preferred embodiment, the at least one polymethine dye comprised in the nanostructured delivery system mediates targeted transport of the nanostructured delivery system into adenocarcinoma stem cells. As used in the invention, the term "adenocarcinoma stem cell" refers to a cell, which originates from a normal stem or a progenitor cell which has undergone a mutation. One or more mutations may occur at any point during the normal developmental progression from a stem cell to progenitor cell to mature cell. The mutated cell thereby gives rise to an entity that is functionally defined, i.e. the adenocarcinoma stem cell : the stem cell may differentiate into any cell of the respective adenocarcinoma lineage carrying the defect. such stem cells have been described in the context of lung cancer, gastrointestinal cancer and prostate cancer.

In a preferred embodiment, the adenocarcinomatous cancer to be treated by using the nanostructured delivery system of the invention may be a prostate cancer or a colon cancer.

In the nanostructured delivery system the at least one polymethine dye may be a symmetrical or asymmetrical polymethine of the general structure 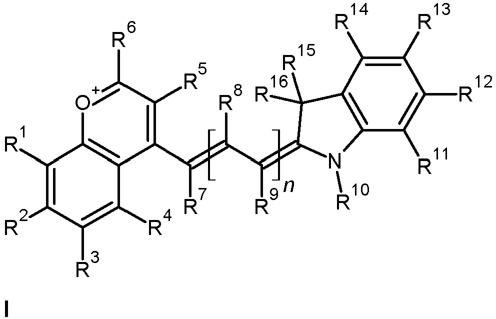 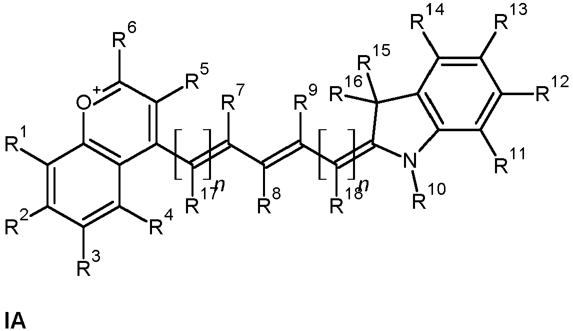 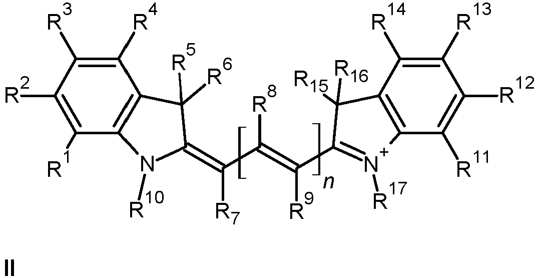 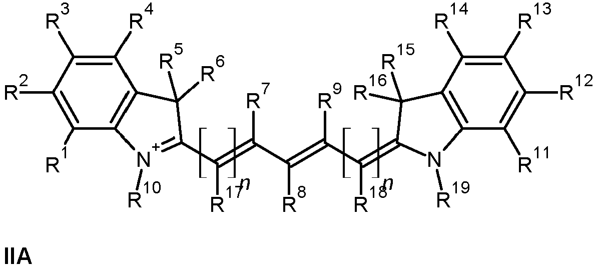
a. n stands for the numerical value 1, 2 or 3;
b. R1-R19 may be the same or different and maybe hydrogen or deuterium, one or more alkyl, tert-alkyl, cycloalkyl- (the "alkyl" and "cycloalkyl" radicals also include olefinic structures) or aryl, carboxyaryl, dicarboxyaryl, heteroaryl or heterocycloaliphatic radicals, alkyloxy, acyloxy, alkylmercapto, arlyoxy, arylmercapto, heteroaryloxy, heteroarylmercapto groups, a hydroxyl, halogen, nitro, amino, aminoalkyl, aminoaryl, aminoacyl, halogenated alkyl, formyl, azido, thio(iso)cyanato, (iso)cyanato, carbamate, thiocarbamate, urea, thiourea, guanidine, sulfonamido, or cyano group, an alkyl-substituted or cyclic amine function and/ or two ortho-position radicals, e.g., R3 and R4, R13 and R14 and/or R1 and R2 and R11 and R12 and/or R7 and R9 together may form an additional aromatic, heteroaromatic, aliphatic or heteroaliphatic ring,
c. at least one of the R1 -R19 substituents has a solubilizing and/or ionizable or ionized substituent such as SO₃⁻, (-SO₃H), PO₃²⁻, COOH, OH or NR₃⁺, cyclo-dextrins or sugar, which determines the hydrophilic properties of these polymethine dyes, wherein this substituent may also be bound to the polymethine dye by a spacer group, and
d. at least one of the R1-R19 substituents has a reactive group (linker) such as isocyanates, isothiocyanates, hydrazines, amines, mono- and dichloro- or mono- and dibromotriazines, aziridines, epoxides, sulfonyl halides, acid halides, carboxylic anhydrides, N-hydroxy-succinimide esters, imido esters, carboxylic acids, glyoxal, aldehyde, maleimide or iodacetamide and phosphoramidite derivatives or azides, alkynes or olefins, wherein this substituent may also be bound to the polymethine dye by a spacer group.
e. the aromatic, heteroaromatic, aliphatic or heteroaliphatic spacer group consists of structural elements such as [(CH₂)ₐ - Y- (CH₂)_{b}]_{c} or [(C₆H₄)ₐ - Y- (C₆H₄)_{b}]_{c}, where Y may be the same or different and comprises CR₂-, O-, S-, -SO₂, SO₂NH-, NR-, COO- or CONR functions, wherein it is bound to one of the R1 - R19 substituents, and a.) and b.) may be the same or different and have numerical values of 0-18 and numerical values for c of 0-18,
f. the R7, R8 and R9 substituents and/or R17 and R18 may also be present 2 times, 3 times, 4 times or 5 times, and these may be the same or different,
g. R15 and R16 and/or R5 and R6 may form an additional alicyclic or heterocyclic ring system.

It is especially preferred if at least one of the R1 -R19 substituents has a solubilizing and/or ionizable or ionized substituent such as SO₃⁻, (-SO₃H), PO₃²⁻, COOH, OH, cyclo-dextrins or sugar, which determines the hydrophilic properties of these polymethine dyes, wherein this substituent may also be bound to the polymethine dye by a spacer group. Moreover, it is particularly preferred, if the aromatic, heteroaromatic, aliphatic or heteroaliphatic spacer group consists of structural elements such as [(CH₂)ₐ - Y- (CH₂)_{b}]_{c} or [(C₆H₄)ₐ - Y- (C₆H₄)_{b}]_{c}, where Y may be the same or different and comprises CH₂-, O-, S-, -SO₂, SO₂NH-, NH-, COO- or CONH or alkylated analogues, where H atoms are substitued by alkyl chains C-alk₂, O-, S-, -SO₂, SO₂N-alk, N-alk, COO- or CON-alk functions, wherein it is bound to one of the R1 -R19 substituents, and a.) and b.) may be the same or different and have numerical values of 0-18 and numerical values for c of 0-18.

In a preferred embodiment, the at least one polymethine dye comprised in the nanostructured delivery system may be characterized by at least one molecular descriptor. A "molecular descriptor" (or a "molecular predictor") refers to a value or a coefficient relating to a defined property of a molecule of interest, wherein the value is determined from a symbolic representation of said molecule. Thus, a molecular descriptor allows the representation of chemical information of a molecule in a computer-interpretable vector of numbers. In contrast, actual physicochemical properties of a given molecule are derived from experimental measurements. In the context of the present invention, the at least one molecular descriptor may be selected from the group comprising topological polar surface area, logP value, number of atoms, molecular weight, number of oxygen and nitrogen atoms, number of OH and NH (H-bond donors), number of sulfonyl residues or other functional groups contributing to a negative charge at pH 7.4, number of rotatable bonds, molecular volume. The topological polar surface area, which is defined as the surface sum over all polar atoms or molecules, primarily oxygen and nitrogen (also including their attached hydrogen atom), is between 50 and 200 Å², preferably between 100 to 150 Å² and even more preferred about 130 Å². The logP coefficient, which is well-known as one of the principal parameters for the estimation of lipophilicity of chemical compounds (determining their pharmacokinetic properties), is between -3 and 7, preferably between 4 and 6.5. The number of atoms is between 30 and 70, preferably between 40 and 60. The molecular weight is between 400 and 1100g/mol, preferably between 500 and 800 and most suitably in the range between 650 and 750 g/mol. The number of oxygen and nitrogen atoms is between 4 and 20, preferably between 7 and 12, whereas the number of OH and NH (H-bond donors) is between 1 and 8, preferably between 3 and 6. The number of sulfate residues or other functional groups contributing to a negative charge at pH 7.4, is between 0 and 5, preferably between 0 and 3 and most preferred between 1 and 2. The number of rotatable bonds, which is the number of bonds allowing free rotation around themselves (defined as any single bond, not in a ring, bound to a nonterminal heavy atom), is between 5 and 30, preferably between 10 and 20. The molecular volume, which is the volume occupied by one mole of a substance (chemical element or chemical compound) at a given temperature and pressure (equal to molar mass, M, divided by mass density, ρ), is between 500 and 1100 Å³, preferably between 600 and 800 Å³.

In a preferred form, the polymethine dye is a class I dye with n=1 and R1, R2 and R3 are heteroxyclic fused with an octahydro-2H-quinolizine to form a 2,3,6,7-tetrahydro-1H,5H-benzo(ij)quinolizine (aka Julolidine) moiety, and R6 is tert. butyl, R15 and R16 is methyl, R10 is hexanoic acid residue and R13 is a sulfonic acid residue. DY635:
In a preferred form, the polymethine dye is a class I dye with n=1 and wherein R1, R2 and R3 are heteroxyclic fused with an octahydro-2H-quinolizine to form a 2,3,6,7-tetrahydro-1H,5H-benzo(ij)quinolizine (aka Julolidine) moiety, and R6 is tert. butyl, R15 is methyl and R16 butanoic acid residue, R10 is propane-1-sulfonic acid and R13 is a sulfonic acid residue. 636
In a preferred form, the polymethine dye is a class I dye with n=1 and R2 is NH2, R6 is tert. butyl, R15 and R16 is methyl and R10 is hexanoic acid residue and R13 is a sulfonic acid residue. 615
In a preferred form, the polymethine dye is a class I dye with n=1, and wherein R2 and R3 are heteroxyclic fused with 3-[2,2-dimethyl-4-(sulfomethyl)pyridin-1(2H)-yl]propane-1-sulfonic acid to form a 3-[2,2-dimethyl-4-(sulfomethyl)quinolin-1(2H)-yl]propane-1-sulfonic acid moiety, R6 is phenyl, R15 is methyl and R16 butanoic acid residue, R10 is propane-1-sulfonic acid and R13 is a sulfonic acid residue.

In a preferred form, the polymethine dye is a class la dye with n=1, and wherein R2 is diethylamino, R6 is tert. butyl, R15 and R16 is methyl, R10 is hexanoic acid residue and R13 is a sulfonic acid residue.

In a preferred form, the polymethine dye is a class la dye with n=1, and wherein R2 and R3 are heteroxyclic fused with 1-ethyl-2,2,4-trimethyl-1,2-dihydropyridine to form a 1-ethyl-2,2,4-trimethyl-1,2-dihydroquinoline moiety, R15 and R16 is methyl, R6 is tert. butyl, R10 is hexanoic acid residue and R13 is a sulfonic acid residue.

In a preferred form, the polymethine dye is a class la dye with n=1 and R1, R2 and R3 are heteroxyclic fused with an octahydro-2H-quinolizine to form a 2,3,6,7-tetrahydro-1H,5H-benzo(ij)quinolizine (aka Julolidine) moiety, and R6 is tert. butyl, R15 is methyl, R16 is butanoic acid, R10 is propane-1-sulfonic acid and R13 is a sulfonic acid residue.

In a preferred form, the polymethine dye is a class I dye, wherein R2 is diethylamino, R6 is tert. butyl, R15 and is methyl, R10 is hexanoic acid and R13 is a sulfonic acid residue.

In a preferred form, the polymethine dye is a class I dye with n=1 and wherein R2 is diethylamino, R6 is tert. butyl, R15 is methyl, R16 is butanoic acid, R10 is propane-1-sulfonic acid and R13 is a sulfonic acid residue.

In a preferred form, the polymethine dye is a class IIa dye with n=1, and wherein R3 and R13 is a sulfonic acid residue, R5, R6, R15 and R16 are methyl groups, R10 is a 2-methoxyethyl residue and R17 is hexanoic acid.

In a preferred form, the polymethine dye is a class IIa dye with n=1, and wherein R3 and R13 is a sulfonic acid residue, R5, R6 and R15 are methyl groups, R16 is a propane-1-sulfonic acid, R10 is a 2-methoxyethyl residue and R17 is hexanoic acid.

In a preferred form, the polymethine dye is a class IIa dye with n=1, and wherein R5, R6, R15 and R16 are methyl groups, R10 is a 2-methoxyethyl residue, R17 is hexanoic acid and R13 is a sulfonic acid residue.

In a preferred form, the polymethine dye is a class la dye with n=1, and wherein R3, R4 and R13, R14 are benzo-fused, R5, R6, R15, R16 and R10 are methyl groups, R17 is a hexanoic acid.

In a preferred form, the polymethine dye is a class IIa dye with n=2, and wherein R7 and R9 are fused to a cyclohexene moiety, R8 is phenoxy, R3 and R4 are benzo-fused, R15 and R16 are methyl, R10 is hexanoic acid and R17 is butane-1-sulfonic acid.

In a preferred form, the polymethine dye is a class la dye with n=1, and wherein R2 and R3 are heteroxyclic fused with 3-[2,2-dimethyl-4-(sulfomethyl)pyridin-1(2H)-yl]propane-1-sulfonic acid to form a 3-[2,2-dimethyl-4-(sulfomethyl)quinolin-1(2H)-yl]propane-1-sulfonic acid moiety, R6 is phenyl, R15 is methyl and R16 butanoic acid residue, R10 is propane-1-sulfonic acid and R13 is a sulfonic acid residue.

In a further embodiment, the at least one polymethine dye in the nanostructured delivery system of the invention may be selected from the group consisting of DY635, DY730, DY736, DY615, DY636, DY731, DY647P1, DY648P1, CY-E10 derivatives, CY5.5 derivatives, DY630, IRDye800; DY778.

The nanostructured delivery system comprises additionally at least one active pharmaceutical ingredient; the at least one active pharmaceutical ingredient is selected from the group comprising nucleic acids, such as small RNAs like miRNA, siRNA, or DNAs like plasmids, oligonucleotides, aptamers, DNAzymes; proteins; protacs (proteolysis targeting chimeras), corticosteroids; cytostatics, anti-metabolites; intercalating substances; antibodies; interferons; contrast agents; protein kinase inhibitors, such as tyrosine kinase inhibitors or phosphatidylinositol-3-kinase inhibitors; antibiotics; antifungals; antivirals; anti-inflammatory drugs such as COX inhibitors; glucocorticoids; cell growth inhibitors; mitochondrial uncoupling agents; protein biosynthesis inhibitors; inhibitors of energy metabolism; respiratory chain inhibitors; growth factors inhibitors.

In a second aspect, the invention relates to a pharmaceutical composition as defined in claim 6. In a preferred embodiment of the pharmaceutical composition, the composition is adapted specifically for inhalation.

In a third aspect, the invention relates to a method for targeted transport into adenocarcinoma tissue and/or an adenocarcinoma cell as defined in claim 8. In a preferred embodiment of the inventive method, the neoplastic tissue is a prostate tissue.

In a fourth aspect, the invention relates to the use of the nanostructured delivery system as described above or to the use of the pharmaceutical composition as described above as defined in claim 10.

Specific preferred embodiments of the invention will become evident from the following more detailed description of certain preferred embodiments and the claims.

### Brief description of the figures

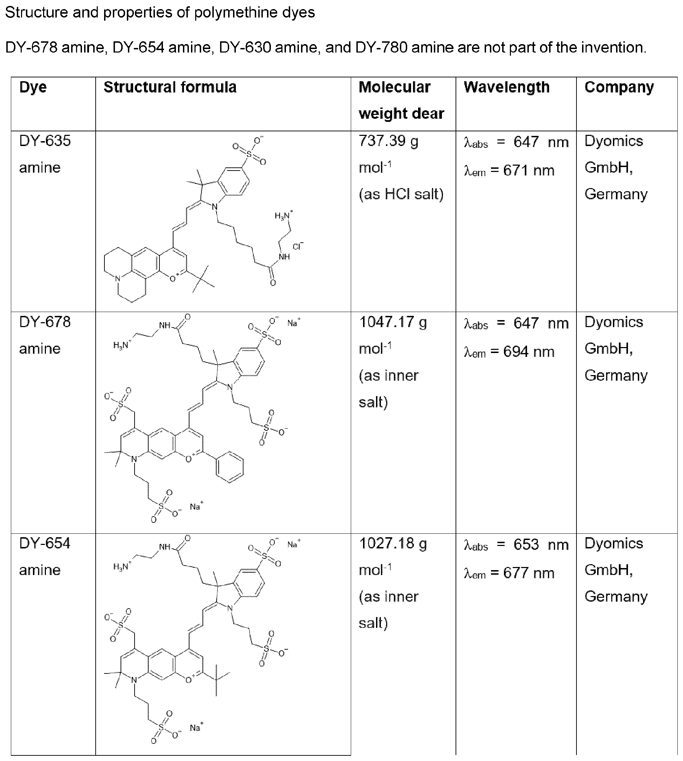

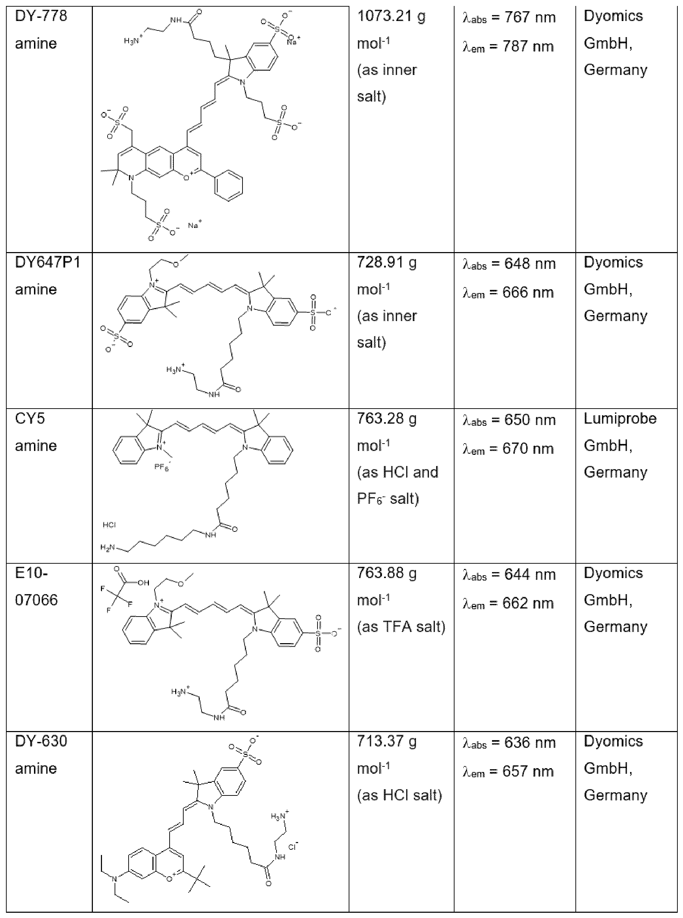

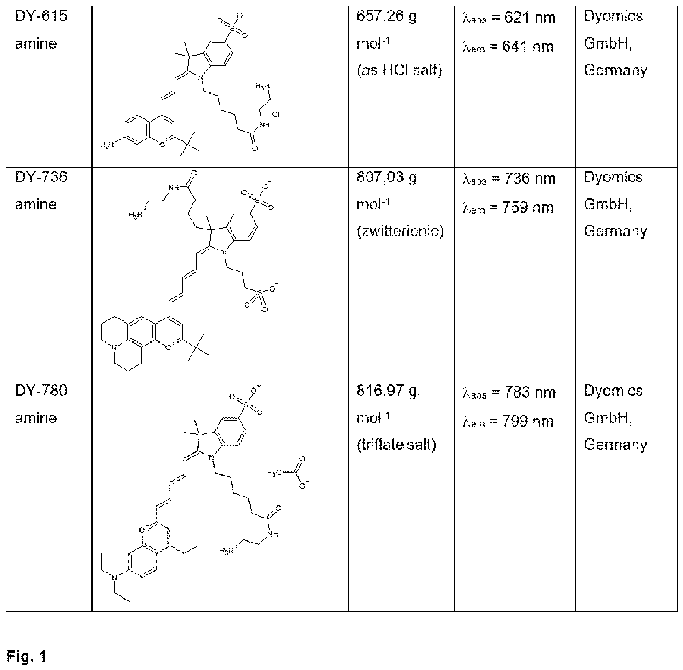

**Figure 1****.** Overview of polymethine dyes grouped into different classes according to their structure and summary of their physicochemical properties.

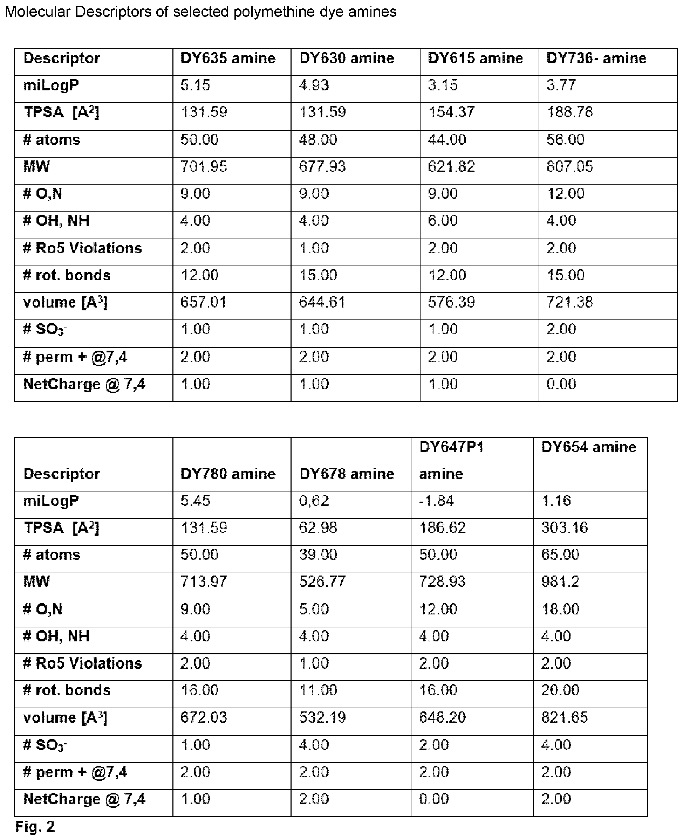

**Figure 2****.** Selected molecular descriptors of the polymethine dye amines of Figure 1. Molecular descriptors were determined at pH = 7.4 with sulfonic acid residues deprotonated and primary aliphatic amines protonated. Counter ions were neglected)

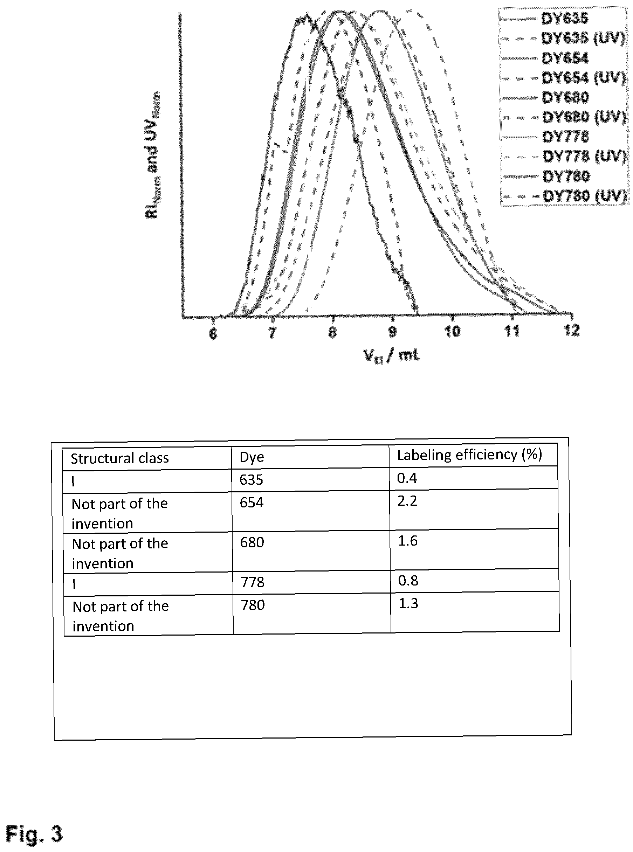

**Figure 3****.** Different dyes may be coupled to PLG using the procedure as described in example 2. In particular, the polymer may be bound covalently to an amine-functionalized dye on the basis of its active carboxylic acid group. Two class I dyes (DY 680, DY 780- not part of the invention) and four class II dyes (DY 630, DY 635, DY 654 (not part of the invention), DY 778) serve as examples. The coupling of each dye-moiety was monitored via size exclusion chromatography (SEC) utilizing UV and refectory index (RI) detection.

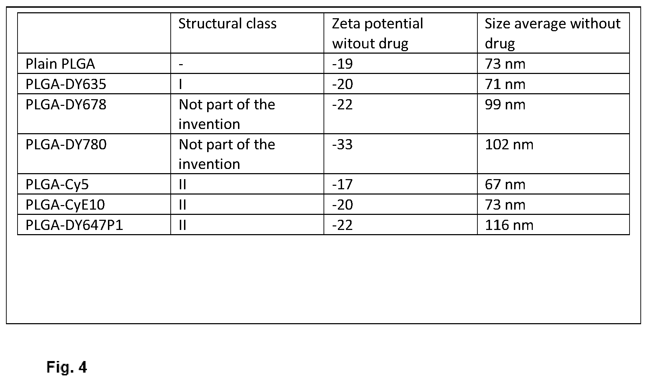

**Figure 4****.** Nanoparticles of the respective polymethine dye conjugated PLGA (e.g., DY635-PLGA) were produced with constant parameters and characterized utilizing different essays addressing, e.g., size, charge, etc. of the synthesized particles. For the determination of size, dynamic light scatter was used. For the determination of the surface charge (zeta potential), a Zeta-Sizer was utilized in order to determine the electrophoretic signal in a charge determination assay.

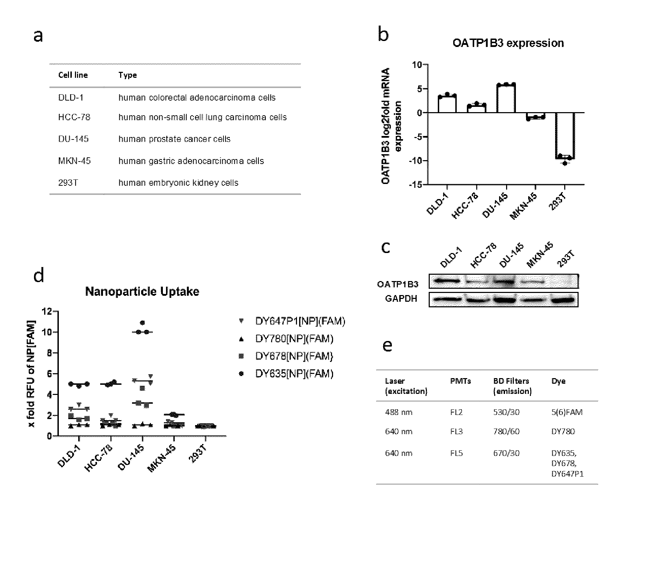

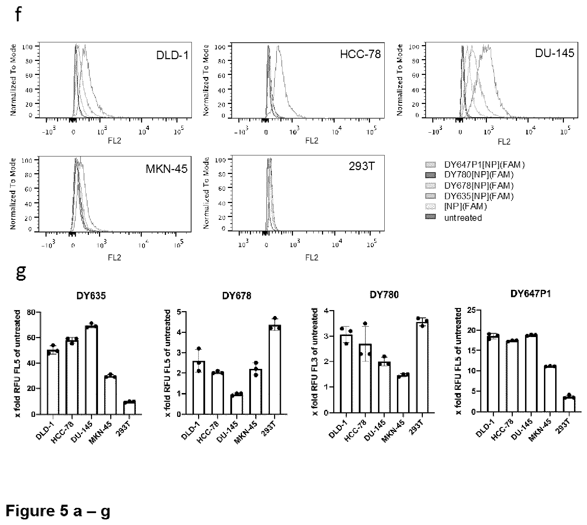

**Figure 5****.** Four adherent tumor cell lines DLD-1, HCC-78, DU-145 and MKN-45 as well as 293T cells as negative control were used (a). OATP1B3 mRNA expression as well as protein levels were detected by RT-qPCR and Western blot, respectively (b, c). Cells were trypsinized and washed twice with PBS/1%FCS. NP stocks were diluted to 50 µg/mL in PBS/1%FCS. Both cells and NP were prewarmed at 37°C for at least 10 min. 5 x 10⁵ cells were then incubated with NP in 200 µL total volume at 37°C for 5 min and then fixed with 4% paraformaldehyde and washed twice with PBS. Subsequently cells were measured on a BD LSRFortessa. NP uptake was determined by the emission of cargo dye 5(6) carboxyfluorescein (FAM) of NPs relative to non-functionalized NP (NP[FAM]) (d, e, f). Dye incubation was performed equally to NP protocol with 100 nM dye concentration and measured afterwards on a BD LSRFortessa (e, g).

### Detailed description of the invention

The particulars shown herein are by way of example and for purposes of illustrative discussion of the preferred embodiments of the present invention only and are presented in the cause of providing what is believed to be the most useful and readily understood description of the principles and conceptual aspects of various embodiments of the invention. In this regard, no attempt is made to show structural details of the invention in more detail than is necessary for the fundamental understanding of the invention, the description taken with the drawings and/or examples making apparent to those skilled in the art how the several forms of the invention may be embodied in practice.

The following definitions and explanations are meant and intended to be controlling in any future construction unless clearly and unambiguously modified in the following examples or when application of the meaning renders any construction meaningless or essentially meaningless. In cases where the construction of the term would render it meaningless or essentially meaningless, the definition should be taken from Webster's Dictionary, 3rd Edition or a dictionary known to those of skill in the art, such as the Oxford Dictionary of Biochemistry and Molecular Biology (Ed. Anthony Smith, Oxford University Press, Oxford, 2004).

### Example 1: Synthesis of Functionalized Polymers

The synthesized nanoparticles are based on the hydrophobic polymer poly (lactic co-glycolic acid) (PLGA), which is biocompatible and biodegradable. This polymer may be bonded covalently to an amine-functionalized dye, as described previously (EP2848262A1). In brief, due to the acid-terminated group of the dye molecule coupling reagents such as EDC (1-ethyl-3- (3-dimethylaminopropyl) carbodiimide) may be used. Here polymethine dyes DY635, DY630, DY615, DY736 were used for most experiments (see **FIG.1**). Every 100th polymer chain was functionalized. Specific polymethine dyes were selected according to predetermined molecular descriptors; in **FIG.2****,** molecular descriptors of the polymethine dye amines of FIG. 1 are depicted. Molecular descriptors were determined at pH = 7.4 with sulfonic acid residues deprotonated and primary aliphatic amines protonated. Counter ions were neglected. Molecular descriptor where selected from the group comprising, logP value, topological polar surface area, number of atoms, molecular weight, number of oxygen and nitrogen atoms, number of OH and NH (H-bond donors), number of sulfate residues or other functional groups contributing to a negative charge at pH 7.4, number of rotatable bonds, molecular volume. Therein, the topological polar surface area is defined as the surface sum over all polar atoms or molecules, primarily oxygen and nitrogen (also including their attached hydrogen atom). The logP coefficient is well-known as one of the principal parameters for the estimation of lipophilicity of chemical compounds (determining their pharmacokinetic properties). The number of rotatable bonds is the number of bonds allowing free rotation around themselves (defined as any single bond, not in a ring, bound to a nonterminal heavy atom). The molecular volume is the volume occupied by one mole of a substance (chemical element or chemical compound) at a given temperature and pressure (equal to molar mass, M, divided by mass density, ρ).

### Example 2: Production of nanoparticles

After functionalization of the polymers, nanoparticles were prepared by means of simple (A) and double (B) emulsions, as described previously (EP 2848262A1). In brief, high-frequency ultrasound was used for forming nanoscale particles in the presence of surface-active substances (surfactant/s). Hydrophobic polymers were dissolved in ethyl acetate. The polymer suspension was added to water with surfactant and nanoparticles were formed by ultrasound (A). If hydrophilic substances were to be included, the hydrophilic substance was dissolved in water, added to the polymer in ethyl acetate and ultrasonicated. Subsequently, water mixed with surfactant was added and nanoparticles were formed by ultrasound (B). The nanoparticles thus obtained were then stirred under an air flow until all the organic solvent (ethyl acetate) had evaporated and the particles were thus stable in water. In order to remove the excess surfactant, the nanoparticles were washed at least twice thoroughly with ultrapure water. Finally, the particles were lyophilized and their mass determined.

### Example 3: Characterization of the Nanoparticles

Nanoparticles of of various DY-conjugated PLGA particles were prepared; for example, DY635-conjugated PLGA (DY635-PLGA-NP), DY654-conjugated PLGA (DY654-PLGA-NP - not part of the invention), DY678-conjugated PLGA (DY678-PLGA-NP - not part of the invention), DY680-conjugated PLGA (DY680-PLGA-NP - not part of the invention), DY778-conjugated PLGA (DY778-PLGA-NP), and DY780-conjugated PLGA (DY780-PLGA-NP - not part of the invention), Cy5-conjugated PLGA (Cy5-PLGA-NP), CyE10-conjugated PLGA (CyE10-PLGA-NP), and DY647P1-conjugated PLGA (DY647P1-PLGA-NP) were prepared and reproduced with constant parameters. The assays used are listed below:
Size: Measurement of the size of the various nanostructured carrier systems dissolved in deionized water by dynamic light scattering (e.g. Zetasizer (Malvern Instruments GmbH)) or electron micrographs (see **FIG.4****).** Alternatively, size was determined relying on size exclusion chromatography (SEC) combining UV and refectory index (RI) detection. SEC elugrams of above exemplary dyes are depicted in **FIG. 3****.**
Shape: Determination of the shape by electron micrographs.
Charge: Measurement of the various nanostructured carrier systems dissolved in deionized water at the Zetasizer (Malvern Instruments GmbH) by determination of the electrophoretic signal (ζ-potential, surface charge), see **FIG.4****.**
Endotoxins: Endotoxin measurement by LAL chromogenic assay (Guilfoyle, DE et al., Evaluation of a chromogenic procedure for use with the Limulus lysate assay of bacterial endotoxin in drug products. J Parenter Sci Technol, 1985. 39 (6): pp. 233-6).
Hemolysis: Measurement of the hemoglobin concentration of erythrocytes incubated with the particles in physiological buffer for 1 h. If the erythrocyte membrane is damaged, the measurable hemoglobin concentration in the supernatant increases.
Aggregation: Measurement of the absorption of erythrocytes incubated with the polymers in physiological buffer. Cell aggregate samples show lower absorption than homogeneously distributed, non-aggregated cells.

### Example 4: RNA isolation

InnuPREP RNA Mini Kit (Analytik Jena AG, Jena, Germany) was used to isolate RNA from cultured cell lines. The procedure was carried out analogously to the manufacturer's instructions. Briefly, the cells were washed once with 10 ml of D-PBS after removal from the culture flask and then centrifuged at 1000 rpm for 5 min at RT. The pellet of max. 5 x 10⁶ cells were lysed with 450 µl Lysis Solution RL containing guanidinium thiocyanates. The lysate was then pipetted onto the Spin Filter D placed in a 2 ml receiver tube and centrifuged at 12,000 rpm for 2 min at RT, whereby the selective removing of gDNA was performed. 400 µl of 70% ethanol were added to the homogenate and mixed well by repetitive pipetting. In the following, the entire volume was placed on the Spin Filter R placed in a 2 ml receiver tube and centrifuged for 2 min at 12,000 rpm at RT, whereby the selective binding of RNA was carried out on the filter. The tube was discarded and the Spin Filter R placed on a new 2 ml receiver tube. 500 µl of Washing Solution HS were then added to the filter and centrifuged for 1 min at 12,000 rpm at RT. The Spin Filter R was then placed again on a new 2 ml receiver tube, 700 µl of Washing Solution LS were added to the filter and centrifuged for 3 min at 12000 rpm at RT. The RNeasy spin column was then placed on a 1.5 ml elution tube with lid. For elution, 30 µl of RNase free water were pipetted onto the Spin Filter R, incubated at RT for 1 min and centrifuged at 8,000 rpm for 1 min. The RNA concentration of the eluate was then determined with NanoDrop and the cDNA synthesis was carried out directly afterwards.

### Example 5: cDNA synthesis

For efficient cDNA synthesis, a concentration of 500 ng µL⁻¹ RNA was initially prepared by diluting the RNA used by RNAse free water (Analytik Jena AG). In a 1.5 mL Eppendorf tube in each case, 7.7 µL of RNA elution were mixed with 4 µl of dNTP mix (Fermentas) and 1 µL of random hexamer primer (Thermo Scientific) by pipetting up and down and then incubated for 5 min at 65°C in the thermocycler (Eppendorf, Thermomixer comfort). Subsequently, 4 µL of reaction buffer (5 × First Strand Buffer, Invitrogen), 2 µL of 0.1 M DTT (Invitrogen) and 0.5 µL of RNaseOut ^{™} Recombinant (40 U ml⁻¹ Ribonuclease Inhibitor) were added to the sample by pipetting up and down and the sample was incubated for 2 min at 37°C in a thermocycler. The reaction was then supplemented with 0.8 µL of M-MLV reverse transcriptase (200 U µL⁻¹, Invitrogen), mixed well by vortexing and briefly centrifuged (table centrifuge, Eppendorf 5415 D). The cDNA synthesis was subsequently carried out in the thermomixer (Eppendorf). Annealing was initially carried out at 25°C for 10 min, the following extension for 60 min at 37°C and inactivation of the reverse transcriptase for 15 min at 70°C. The resulting cDNA was then stored at -20°C.

**Table E5: Reagents for cDNA synthesis**

| **Reagent** | **Concentration** | **Company** |
|---|---|---|
| M-MLV reverse transcriptase | 200 U µL⁻¹ | Invitrogen, Karlsruhe, Germnay |
| Random-Hexamer Primer | 0.2 µg µL⁻¹ | Thermo Scientific, Darmstadt, Germany |
| dNTP-Mix | 2.5 mM | Fermentas, St. Leon-Rot, Germany |
| Reaction buffer | 5 fold | Invitrogen |
| DTT | 0.1 M | Invitrogen |
| RNaseOUT^{™} | 40 U µL⁻¹ | Invitrogen |

### Example 6: Quantitative Real-Time PCR (qRT-PCR)

For reliable detection of gene expression alteration, the qRT-PCR method may be employed, as described in the following. For carrier protein expression analysis using density plots as depicted in **FIG. 5b****,** all operations were performed under an ultra-violet sterilizing PCR workstation (Peqlab, Biotechnology GmbH). A reaction solution with a total volume of 20 µL (10 µL of LightCycler^{®} 480 SYBR Green I Master Mix (Roche Diagnostics) + 8 µL sterile PCR water + 1 µL of 1:10 dilute primer mix + 1 µL of cDNA) was added to a 96-well plate (twin.tec PCR Plate 96, semi-skirted, blue, Eppendorf). The wells were then closed with flat cap strips (Eppendorf) and the 96-well plate was briefly centrifuged (Centrifuge 5810 R, Eppendorf). PCR was performed on Mastercycler^{®} ep gradient S realplex4 (Eppendorf AG, Hamburg, Germany). Between final elongation and cooling, the products melted slowly to 95°C in order to assess the quality of the PCR product with the melting curve. Samples were run on 96-well plates in triplicates and gene expression was normalized using ΔCₜ method against housekeeping gene β-glucuronidase (β-GUS). Gene expression level of suspension cell lines was compared to that of HepaRG cells using the 2^{-ΔΔCt} method.

**Table E6.1: Parameters of the PCR steps**

| **PCR step** | **Duration** | **Temperature** | **Repitition** |
|---|---|---|---|
| Denaturation and activation of *Taq*-polymerase | 10 min | 95°C | |
| Denaturation | 15 sec | 95°C | 43 x |
| Primer Annealing | 30 sec | 60.2 - 63.1°C | |
| Elongation | 25 sec | 72°C | |
| Final Elongation | 20 sec | 72°C | |
| Melting | 25 min | 60 - 95°C gradient | |
| Cooling | ∞ | 4°C | |

**Table E7.2: Sequences of PCR primers**

| **Gene** | **Primer** | **Sequence in 5'→ 3' direction** | **Annealing temperature** |
|---|---|---|---|
| SLCO1B3 (OATP1B3) | fwd | GTCCAGTCATTGGCTTTGCA CAACCCAACGAGAGTCCTTAGG | 63.1°C |
| | rev | | |
| β-GUS | fwd | AGAAACGATTGCAGGGTTTCAC CCGAGTGAAGATCCCCTTTTTA | 62.0°C |
| | rev | | |

### Example 7: Dye and nanoparticle experiments

Four adherent tumor cell lines DLD-1, HCC-78, DU-145 and MKN-45 as well as 293T cells as negative control were used. Of each dye 100 nM dilutions were prepared by diluting the stock solution with D-PBS. Subsequently, 5 × 10⁵ cells from the were trypsinized and pipetted in 100 µl in 1.5 ml of tube and stored both at 37°C in the thermocycler (Thermomixer comfort, Eppendorf) and at 4°C on ice for at least 10 min. The cells were then incubated simultaneously at 37°C and 4°C with 100 µl of the different dye concentration for 5 min in the dark and then fixed for 15 with 4% paraformaldehyde. Samples were centrifuged at 1000 rpm for 5 min (Eppendorf Centrifuge 5415 C) and washed twice with 1 ml D-PBS + 1% BSA after discarding the supernatant. The pellet was then resuspended in 500 µl D-PBS + 1% BSA, transferred to FACS tubes and measured after short vortexing at BD LSRFortessa. For nanoparticle (NP) experiments 5 × 10⁵ cells per 100 µl were set with pre-heated RPMI + 10% FCS and 100 µl per well were sown in two 24-well plates so that the nanoparticle incubations could be carried out at 4°C and 37°C. By addition of MilliQ water dilutions of 100 µg ml⁻¹ were prepared from NP stock (10 mg ml⁻¹ each) so that after application of 100 µl to one cell suspension per well, an incubation concentration of 50 µg ml-1 could thus be achieved. Cells were incubated for 5 min in the dark at 4°C on ice and 37°C on the thermomixer and subsequently transferred to FACS tubes in a volume of 250 µl of D-PBS and immediately measured at BD LSRFortessa.

## Claims

1. Nanostructured delivery system comprising at least one polymer and/or at least one lipid, at least one polymethine dye, and at least one active pharmaceutical ingredient for use in treating a subject suffering from an adenocarcinoma, wherein the adenocarcinoma is a cancer selected from the group consisting of gastrointestinal cancer, lung cancer, prostate cancer, colon cancer, and breast cancer, and comprises adenocarcinoma cells with a gene expression alteration in one or more *SLCOL1* genes, wherein gene expression alteration refers to an alteration in protein synthesis of the respective gene, which is present in a neoplastic cell of a tissue but not, or not to the same extent, in a normal, non-neoplastic cell of said tissue, wherein the at least one polymethine dye mediates targeted transport of the nanostructured delivery system into said adenocarcinoma cells via the expression product of the one or more SLCO 1 genes, and
wherein the at least one polymethine dye is a polymethine of the general structure 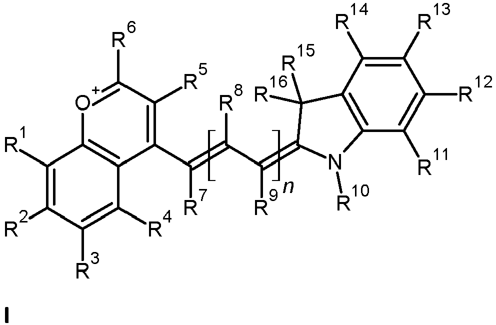 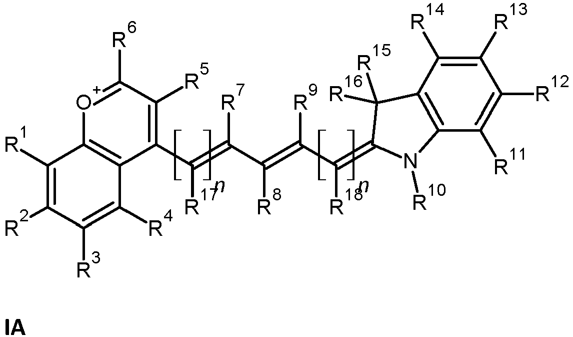 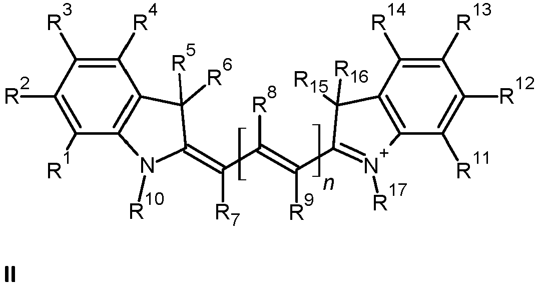 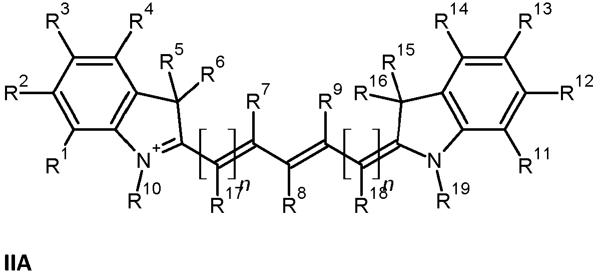
a. n stands for the numerical value 1, 2 or 3;
b. R1-R19 may be the same or different and maybe hydrogen or deuterium, one or more alkyl, tert-alkyl, cycloalkyl- (the "alkyl" and "cycloalkyl" radicals also include olefinic structures) or aryl, carboxyaryl, dicarboxyaryl, heteroaryl or heterocycloaliphatic radicals, alkyloxy, acyloxy, alkylmercapto, arlyoxy, arylmercapto, heteroaryloxy, heteroarylmercapto groups, a hydroxyl, halogen, nitro, amino, aminoalkyl, aminoaryl, aminoacyl, halogenated alkyl, formyl, azido, thio(iso)cyanato, (iso)cyanato, carbamate, thiocarbamate, urea, thiourea, guanidine, sulfonamido, or cyano group, an alkyl-substituted or cyclic amine function and/ or two ortho-position radicals together may form an additional aromatic, heteroaromatic, aliphatic or heteroaliphatic ring,
c. at least one of the R1 -R19 substituents has a solubilizing and/or ionizable or ionized substituent such as SO₃⁻, (- SO₃H), PO₃²⁻, COOH, OH or NR₃+, cyclo-dextrins or sugar, which determines the hydrophilic properties of these polymethine dyes, wherein this substituent may also be bound to the polymethine dye by a spacer group, and
d. at least one of the R1-R19 substituents has a reactive group (linker) such as isocyanates, isothiocyanates, hydrazines, amines, mono- and dichloro- or mono- and dibromotriazines, aziridines, epoxides, sulfonyl halides, acid halides, carboxylic anhydrides, N-hydroxy-succinimide esters, imido esters, carboxylic acids, glyoxal, aldehyde, maleimide or iodacetamide and phosphoramidite derivatives or azides, alkynes or olefins, wherein this substituent may also be bound to the polymethine dye by a spacer group,
e. the aromatic, heteroaromatic, aliphatic or heteroaliphatic spacer group consists of structural elements such as [(CH₂)ₐ - Y- (CH₂)_{b}]_{c} or [(C₆H₄)ₐ - Y-(C₆H₄)_{b}]_{c}, where Y may be the same or different and comprises CR₂-, O-, S-, - SO₂, SO₂NH-, NR-, COO- or CONR functions, wherein it is bound to one of the R1 -R19 substituents, and a.) and b.) may be the same or different and have numerical values of 0-18 and numerical values for c of 0-18,
f. the R7, R8 and R9 substituents and/or R17 and R18 may also be present 2 times, 3 times, 4 times or 5 times, and these may be the same or different, and
g. R15 and R16 and/or R5 and R6 may form an additional alicyclic or heterocyclic ring system; and
wherein the at least one active pharmaceutical ingredient is selected from the group comprising nucleic acids, proteins, protacs (proteolysis targeting chimeras), corticosteroids, cytostatics, anti-metabolites, intercalating substances, antibodies, interferons, contrast agents, protein kinase inhibitors, antibiotics, antifungals, antivirals, anti-inflammatory drugs, glucocorticoids, cell growth inhibitors, mitochondrial uncoupling agents, protein biosynthesis inhibitors, inhibitors of energy metabolism, respiratory chain inhibitors, and growth factors inhibitors.

2. Nanostructured delivery system for the use according to claim 1, wherein the adenocarcinoma comprises adenocarcinoma cells in which said gene expression alteration affects the *SCLO1B3* gene.

3. Nanostructured delivery system for the use according to according to claim 1 or 2, wherein the at least one polymethine dye mediates targeted transport of the nanostructured delivery system into adenocarcinoma stem cells.

4. Nanostructured delivery system for the use according to claim 1, wherein the at least one polymethine dye is characterized at pH7.4 by at least one molecular descriptor selected from the group comprising topological polar surface area, logP value, number of atoms, molecular weight, number of oxygen and nitrogen atoms, number of OH and NH (H-bond donors), number of sulfate residues or other functional groups contributing to a negative charge at pH 7.4, number of rotatable bonds, molecular volume, wherein the topological polar surface area is between 50 and 200 Å², the logP value is between -3 and 7, the number of atoms is between 30 and 70, the molecular weight is between 400 and 1100g/mol, the number of oxygen and nitrogen atoms is between 4 and 20, the number of OH and NH (H-bond donors) is between 1 and 8, the number of sulfate residues or other functional groups contributing to a negative charge at pH 7.4, is between 0 and 5, the number of rotatable bonds is between 5 and 30, the molecular volume is between 500 and 1100 Å³.

5. Nanostructured delivery system for the use according to any one of the preceding claims, wherein the at least one polymethine dye is selected from the group consisting of DY635, DY730, DY736, DY615, DY636, DY731, DY647P1, DY648P1, CY-E10 derivatives, CY5.5 derivatives, DY630, IRDye800; DY778.

6. Pharmaceutical composition comprising a nanostructured delivery system according to any one of claim 1 to 5 as well as suitable excipients and additives for use in treating a subject suffering from an adenocarcinoma, wherein the adenocarcinoma is a cancer selected from the group consisting of gastrointestinal cancer, lung cancer, prostate cancer, colon cancer, and breast cancer, and comprises adenocarcinoma cells with a gene expression alteration in one or more *SLCO1* genes.

7. Pharmaceutical composition for the use according to claim 6, specifically adapted for inhalation.

8. Method for targeted transport into adenocarcinoma tissue and/or an adenocarcinoma cell, wherein the adenocarcinoma is a cancer selected from the group consisting of gastrointestinal cancer, lung cancer, prostate cancer, colon cancer, and breast cancer, and comprises adenocarcinoma cells with a gene expression alteration in one or more *SLCO1* genes, comprising contacting the adenocarcinoma tissue and/or the adenocarcinoma cell *in vitro* with a nanostructured delivery system according to any one of claims 1 to 5 or a pharmaceutical composition according to claim 6 or 7.

9. Method according to claim 8, wherein the adenocarcinoma tissue is a prostate tissue.

10. In vitro use of a nanostructured delivery system according to any one of claims 1 to 5 or use of a pharmaceutical composition according to claim 6 or 7, wherein accumulation of the nanostructured delivery system and/or its components is detectable *in vitro* in the adenocarcinoma tissue and/or the adenocarcinoma cell, wherein the adenocarcinoma is a cancer selected from the group consisting of gastrointestinal cancer, lung cancer, prostate cancer, colon cancer, and breast cancer, and comprises adenocarcinoma cells with a gene expression alteration in one or more *SLCO1* genes, by means of the fluorescence properties of the at least one polymethine dye.

## Patentansprüche

1. Nanostrukturiertes Abgabesystem, aufweisend mindestens ein Polymer und/oder mindestens ein Lipid, mindestens einen Polymethinfarbstoff und mindestens einen aktiven pharmazeutischen Inhaltsstoff zur Verwendung bei der Behandlung eines an einem Adenokarzinom leidenden Patienten, wobei das Adenokarzinom ein Krebs ist, der aus der Gruppe ausgewählt ist, die aus Krebserkrankungen des Magen-Darm-Traktes, Lungenkrebs, Prostatakrebs, Dickdarmkrebs und Brustkrebs besteht, und Adenokarzinom-Zellen mit einer Genexpressionsalteration in einem oder mehreren *SLCO1*-Genen enthält, wobei sich Genexpressionsalteration auf eine Alteration in der Proteinsynthese des jeweiligen Gens bezieht, das in einer neoplastischen Zelle eines Gewebes vorhanden ist, jedoch in einer normalen, nicht-neoplastischen Zelle des Gewebes nicht oder nicht zu demselben Grad vorhanden ist, wobei der mindestens eine Polymethinfarbstoff einen gezielten Transport des nanostrukturierten Abgabesystems in die Adenokarzinom-Zellen über das Expressionsprodukt des einen oder der mehreren *SLCO1*-Gene vermittelt, und
wobei der mindestens eine Polymethinfarbstoff ein Polymethin der folgenden allgemeinen Struktur ist: 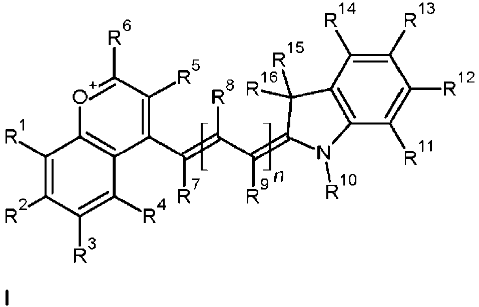 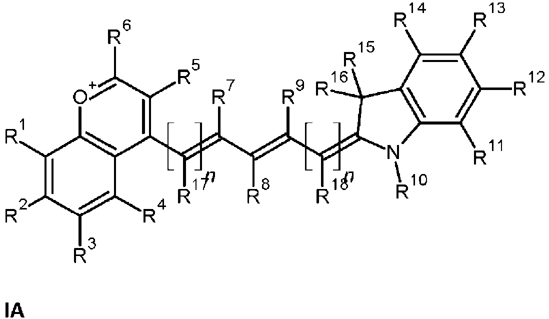 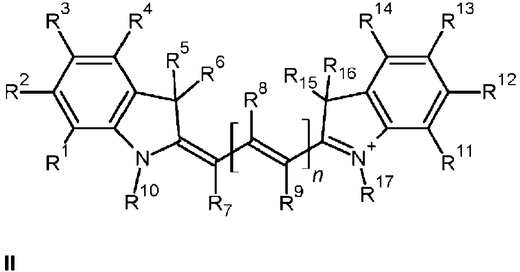 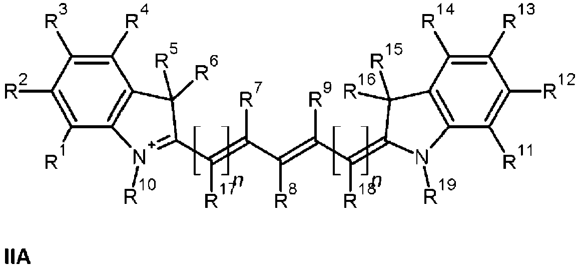
a. wobei n für den numerischen Wert 1, 2 oder 3 steht;
b. R1-R19 gleich oder unterschiedlich sein können und Wasserstoff oder Deuterium, eine oder mehrere Alkyl-, Tert-Alkyl-, Cycloalkyl- (wobei "Alkyl"-und "Cykloalkyl"-Radikale auch Olefinstrukturen beinhalten) oder Aryl-, Carboxyaryl-, Dicarboxyaryl-, Heteroaryl- oder heterocycloaliphatische Radikale, Alkyloxy-, Acyloxy-, Alkylmercapto-, Aryloxy-, Arylmercapto-, Heteroaryloxy-, Heteroarylmercapto-Gruppen, eine Hydroxyl-, Halogen-, Nitro-, Amino-, Aminoalkyl-, Aminoaryl-, Aminoacyl-, halogenierte Alkyl-, Formyl-, Azido-, Thio(iso)cyanat-, (Iso)cyanat-, Carbamat-, Thiocarbamat-, Urea-, Thiourea-, Guanidin-, Sulfonamid- oder Cyano-Gruppe, eine Alkylsubstituierte oder zyklische Amin-Funktion sein kann und/oder zwei Ortho-Positions-Radikale zusammen einen zusätzlichen aromatischen, heteroaromatischen, aliphatischen oder heteroaliphatischen Ring bilden können,
c. mindestens einer der Substituenten R1-R19 einen solubilisierenden und/oder ionisierbaren oder ionisierten Substituenten, wie SO₃₋, (-SO₃H), PO₃²⁻, COOH, OH oder NR₃₊, Cyclo-Dextrine oder Zucker, hat, der die hydrophilen Eigenschaften dieser Polymethinfarbstoffe bestimmt, wobei dieser Substituent auch über eine Spacer-Gruppe an den Polymethinfarbstoff gebunden sein kann, und
d. mindestens einer der Substituenten R1-R19 eine reaktive Gruppe (Linker), wie Isocyanate, Isothiocyanate, Hydrazine, Amine, Mono- und Dichlor- oder Mono- und Dibromtriazine, Aziridine, Epoxide, Sulfonsäurehalogenide, Säure-Halogenide, Carbonsäureanhydride, N-Hydroxysuccinimid-Ester, Imidoester, Carbonsäuren, Glyoxal-, Aldehyd-, Maleimid- oder lodacetamid- und Phosphoramidit-Derivate oder -Azide, Alkine oder Olefine, hat, wobei dieser Substituent auch über eine Spacer-Gruppe an den Polymethinfarbstoff gebunden sein kann,
e. die aromatische, heteroaromatische, aliphatische oder heteroaliphatische Spacer-Gruppe aus Strukturelementen, wie [(CH₂)ₐ - Y- (CH₂)_{b}]_{c} oder [(C₆H₄)ₐ - Y-(C₆H₄)_{b}]_{c}, besteht, wobei Y gleich oder unterschiedlich sein kann und CR₂-, O-, S-, -SO₂, SO₂NH-, NR-, NOO- oder CONR-Funktionen sein kann, wobei es an einen der Substituenten R1-R19 gebunden ist, und a.) und b.) gleich oder unterschiedlich sein können und numerische Werte von 0-18 und numerische Wert für c von 0-18 haben können,
f. die Substituenten R7, R8 und R9 und/oder R17 und R18 auch 2-mal, 3-mal, 4-mal oder 5-mal vorhanden sein können und diese gleich oder unterschiedlich sein können, und
g. R15 und R16 und/oder R5 und R6 ein zusätzliches alizyklisches oder heterozyklisches Ringsystem bilden können; und
wobei der mindestens eine aktive pharmazeutische Inhaltsstoff aus der Gruppe ausgewählt ist, die Nukleinsäuren, Proteine, PROTACS (Proteolysis Targeting Chimeras), Corticosteroide, Cytostatika, Antimetabolite, interkalierende Substanzen, Antikörper, Interferone, Kontrastmittel, Protein-Kinase-Inhibitoren, Antibiotika, Antimykotika, antivirale Medikamente, entzündungshemmende Medikamente, Glucocorticoide, Zellwachstums-Inhibitoren, mitochondriale Entkoppler, Protein-Biosynthese-Inhibitoren, Inhibitoren des Energie-Metabolismus, Inhibitoren der Atmungskette und Wachstumsfaktor-Inhibitoren beinhaltet.

2. Nanostrukturiertes Abgabesystem zur Verwendung gemäß Anspruch 1, wobei das Adenokarzinom Adenokarzinom-Zellen enthält, in denen die Genexpressionsalteration das *SCLO1B3*-Gen betrifft.

3. Nanostrukturiertes Abgabesystem zur Verwendung gemäß Anspruch 1 oder 2, wobei der mindestens eine Polymethinfarbstoff einen gezielten Transport des nanostrukturierten Abgabesystems in Adenokarzinom-Stammzellen vermittelt.

4. Nanostrukturiertes Abgabesystem zur Verwendung gemäß Anspruch 1, wobei der mindestens eine Polymethinfarbstoff bei einem pH-Wert von 7,4 durch mindestens einen Moleküldeskriptor gekennzeichnet ist, der aus der Gruppe ausgewählt ist, die topologische polare Oberfläche, logP-Wert, Anzahl von Atomen, Molekülmasse, Anzahl von Sauerstoff- und Stickstoff-Atomen, Anzahl von OH und NH (H-gebundenen Donoren), Anzahl von Sulfat-Resten oder anderen Funktionsgruppen, die zu einer negativen Ladung bei einem pH-Wert von 7,4 beitragen, Anzahl rotierbarer Bindungen, Molekülvolumen beinhaltet, wobei die topologische polare Oberfläche zwischen 50 und 200 Å² beträgt, der logP-Wert zwischen -3 und 7 liegt, die Anzahl von Atomen zwischen 30 und 70 liegt, die Molekülmasse zwischen 400 und 1100 g/mol liegt, die Anzahl von Sauerstoff- und Stickstoff-Atomen zwischen 4 und 20 ist, die Anzahl von OH und NH (H-gebundenen Donoren) zwischen 1 und 8 liegt, die Anzahl von Sulfat-Resten oder anderen Funktionsgruppen, die zu einer negativen Ladung bei einem pH-Wert von 7,4 beitragen, zwischen 0 und 5 liegt, die Anzahl rotierbarer Bindungen zwischen 5 und 30 liegt, das Molekülvolumen zwischen 500 und 1100 Å³ beträgt.

5. Nanostrukturiertes Abgabesystem zur Verwendung gemäß einem der vorhergehenden Ansprüche, wobei der mindestens eine Polymethinfarbstoff aus der Gruppe ausgewählt ist, die aus DY635, DY730, DY736, DY615, DY636, DY731, DY647P1, DY648P1, CY-E10-Derivaten, CY5.5-Derivaten, DY630, IRDye800; DY778 besteht.

6. Pharmazeutische Zusammensetzung, aufweisend ein nanostrukturiertes Abgabesystem gemäß einem der Ansprüche 1 bis 5, sowie geeignete pharmazeutische Hilfsstoffe und Additive zur Verwendung in der Behandlung eines an einem Adenokarzinom leidenden Patienten, wobei das Adenokarzinom ein Krebs ist, der aus der Gruppe ausgewählt ist, die aus Krebserkrankungen des Magen-Darm-Traktes, Lungenkrebs, Prostatakrebs, Dickdarmkrebs und Brustkrebs besteht, und Adenokarzinom-Zellen mit einer Genexpressionsalteration in einem oder mehreren *SLCO1*-Genen enthält.

7. Pharmazeutische Zusammensetzung zur Verwendung gemäß Anspruch 6, spezifisch angepasst zur Inhalation.

8. Verfahren für den gezielten Transport in Adenokarzinom-Gewebe und/oder eine Adenokarzinom-Zelle, wobei das Adenokarzinom ein Krebs ist, der aus der Gruppe ausgewählt ist, die aus Krebserkrankungen des Magen-Darm-Traktes, Lungenkrebs, Prostatakrebs, Dickdarmkrebs und Brustkrebs besteht, und Adenokarzinom-Zellen mit einer Genexpressionsalteration in einem oder mehreren *SLCO1*-Genen enthält, aufweisend Kontaktieren des Adenokarzinom-Gewebes und/oder der Adenokarzinom-Zelle *in vitro* mit einem nanostrukturierten Abgabesystem zur Verwendung gemäß einem der Ansprüche 1 bis 5 oder einer pharmazeutischen Zusammensetzung gemäß Anspruch 6 oder 7.

9. Verfahren gemäß Anspruch 8, wobei das Adenokarzinom-Gewebe ein Prostata-Gewebe ist.

10. *In*-*vitro*-Verwendung eines nanostrukturierten Abgabesystems gemäß einem der Ansprüche 1 bis 5 oder Verwendung einer pharmazeutischen Zusammensetzung gemäß Anspruch 6 oder 7, wobei eine Ansammlung des nanostrukturierten Abgabesystems und/oder seiner Komponenten in dem Adenokarzinom-Gewebe und/oder der Adenokarzinom-Zelle, wobei das Adenokarzinom ein Krebs ist, der aus der Gruppe ausgewählt ist, die aus Krebserkrankungen des Magen-Darm-Traktes, Lungenkrebs, Prostatakrebs, Dickdarmkrebs und Brustkrebs besteht, und Adenokarzinom-Zellen mit einer Genexpressionsalteration in einem oder mehreren *SLCO1*-Genen enthält, mittels der fluoreszierenden Eigenschaften des mindestens einen Polymethinfarbstoffs *in vitro* erfassbar ist.

## Revendications

1. - Système d'administration nanostructuré comprenant au moins un polymère et/ou au moins un lipide, au moins un colorant polyméthine, et au moins un principe pharmaceutique actif destiné à être utilisé dans le traitement d'un sujet souffrant d'un adénocarcinome, dans lequel l'adénocarcinome est un cancer choisi dans le groupe consistant en le cancer gastro-intestinal, le cancer du poumon, le cancer de la prostate, le cancer du côlon et le cancer du sein, et comprend des cellules d'adénocarcinome présentant une altération d'expression génique dans un ou plusieurs gènes *SLCO1,* l'altération d'expression génique faisant référence à une altération dans la synthèse protéique du gène respectif, qui est présente dans une cellule néoplasique d'un tissu mais non, ou non dans la même mesure, dans une cellule normale, non néoplasique, dudit tissu, ledit au moins un colorant polyméthine médiant un transport ciblé du système d'administration nanostructuré dans lesdites cellules d'adénocarcinome par l'intermédiaire du produit d'expression dudit ou desdits gènes SLCO 1, et
dans lequel ledit moins un colorant polyméthine est une polyméthine de la structure générale : 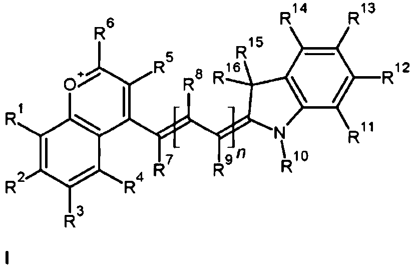 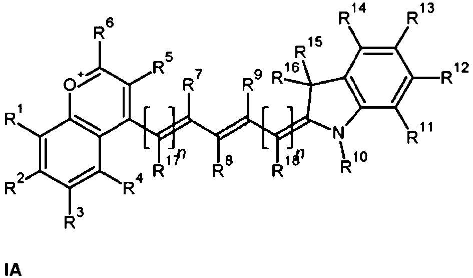 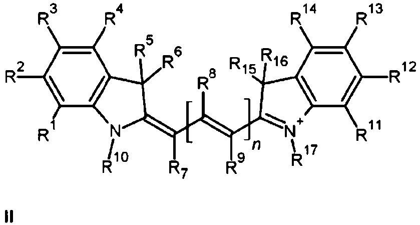 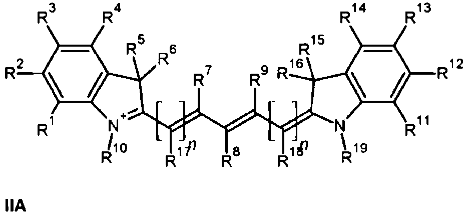
a. n représente la valeur numérique 1, 2 ou 3 ;
b. R1-R19 peuvent être identiques ou différents et peuvent être hydrogène ou deutérium, un ou plusieurs radicaux alkyle, tert.-alkyle, cycloalkyle (les radicaux « alkyle » et « cycloalkyle » comprenant également des structures oléfiniques) ou aryle, carboxyaryle, dicarboxyaryle, hétéroaryle ou hétérocycloaliphatiques, des groupes alkyloxy, acyloxy, alkylmercapto, aryloxy, arylmercapto, hétéroaryloxy, hétéroarylmercapto, un hydroxyle, un halogène, un nitro, un amino, un groupe aminoalkyle, aminoaryle, aminoacyle, alkyle halogéné, formyle, azido, thio(iso)cyanato, (iso)cyanato, carbamate, thiocarbamate, urée, thiourée, guanidine, sulfonamido ou cyano, une fonction amine substituée par un alkyle ou cyclique et/ou deux radicaux en position orthogonale peuvent former ensemble un cycle aromatique, hétéroaromatique, aliphatique ou hétéroaliphatique additionnel ;
c. au moins l'un des substituants R1-R19 a un substituant solubilisant et/ou ionisable ou ionisé tel que SO₃-, (-SO₃H), PO₃²⁻, COOH, OH ou NR₃₊, cyclo-dextrines ou sucre, qui détermine les propriétés hydrophiles de ces colorants polyméthines, ce substituant pouvant également être lié au colorant polyméthine par un groupe espaceur ; et
d. au moins l'un des substituants R1-R19 a un groupe réactif (lieur) tel qu'isocyanates, isothiocyanates, hydrazines, amines, mono- et dichloro- ou mono- et dibromotriazines, aziridines, époxydes, halogénures de sulfonyle, halogénures d'acide, anhydrides carboxyliques, N-hydroxy-succinimide esters, imido esters, acides carboxyliques, glyoxal, aldéhyde, maléimide ou iodacétamide et dérivés de phosphoramidite ou azides, alcynes ou oléfines, ce substituant pouvant également être lié au colorant polyméthine par un groupe espaceur ;
e. le groupe espaceur aromatique, hétéroaromatique, aliphatique ou hétéroaliphatique consiste en des éléments structuraux tels que [(CH₂)ₐ- Y-(CH₂)_{b}]_{c} ou [(C₆H₄)ₐ- Y-(C₆H₄)_{b}]_{c}, où les Y peuvent être identiques ou différents et comprennent les fonctions CR₂-, O-, S-, -SO₂, SO₂NH-, NR-, COO- ou CONR, où il est lié à l'un des substituants R1-R19, et a.) et b.) peuvent être identiques ou différents et avoir des valeurs numériques de 0-18 et des valeurs numériques pour c de 0-18 ;
f. les substituants R7, R8 et R9 et/ou R17 et R18 peuvent également être présents 2 fois, 3 fois, 4 fois ou 5 fois, et ceux-ci peuvent être identiques ou différents ; et
g. R15 et R16 et/ou R5 et R6 peuvent former un système de cycle alicyclique ou hétérocyclique additionnel ; et
dans lequel ledit au moins un principe pharmaceutique actif est choisi dans le groupe comprenant les acides nucléiques, les protéines, les protacs (chimères ciblant la protéolyse), les corticostéroïdes, les cytostatiques, les anti-métabolites, les substances intercalantes, les anticorps, les interférons, les agents de contraste, les inhibiteurs de protéine kinase, les antibiotiques, les antifongiques, les antiviraux, les médicaments anti-inflammatoires, les glucocorticoïdes, les inhibiteurs de la croissance cellulaire, les agents découplants mitochondriaux, les inhibiteurs de la biosynthèse des protéines, inhibiteurs du métabolisme énergétique, inhibiteurs de la chaîne respiratoire et les inhibiteurs des facteurs de croissance.

2. - Système d'administration nanostructuré pour l'utilisation selon la revendication 1, dans lequel l'adénocarcinome comprend des cellules d'adénocarcinome dans lesquelles ladite altération d'expression génique affecte le gène *SCLO1B3.*

3. - Système d'administration nanostructuré pour l'utilisation selon la revendication 1 ou 2, dans lequel ledit au moins un colorant polyméthine médie un transport ciblé du système d'administration nanostructuré dans les cellules souches d'adénocarcinome.

4. - Système d'administration nanostructuré pour l'utilisation selon la revendication 1, dans lequel ledit au moins un colorant polyméthine est caractérisé à pH 7,4 par au moins un descripteur moléculaire choisi dans le groupe comprenant l'aire de surface polaire topologique, la valeur logP, le nombre d'atomes, la masse moléculaire, le nombre d'atomes d'oxygène et d'azote, le nombre de OH et de NH (donneurs de liaisons H), le nombre de restes sulfate ou autres groupes fonctionnels contribuant à une charge négative à pH 7,4, le nombre de liaisons rotatives, le volume moléculaire, l'aire de surface polaire topologique étant entre 50 et 200 Å², la valeur logP étant entre -3 et 7, le nombre d'atomes étant entre 30 et 70, la masse moléculaire étant entre 400 et 1100g/mol, le nombre d'atomes d'oxygène et d'azote étant entre 4 et 20, le nombre de OH et de NH (donneurs de liaisons H) étant entre 1 et 8, le nombre de restes sulfate ou autres groupes fonctionnels contribuant à une charge négative à pH 7,4 étant entre 0 et 5, le nombre de liaisons rotatives étant entre 5 et 30, le volume moléculaire étant entre 500 et 1100 Å³.

5. - Système d'administration nanostructuré pour l'utilisation selon l'une quelconque des revendications précédentes, dans lequel ledit au moins un colorant polyméthine est choisi dans le groupe consistant en DY635, DY730, DY736, DY615, DY636, DY731, DY647P1, DY648P1, les dérivés CY-E10, les dérivés CY5.5, DY630, IRDye800 ; DY778.

6. - Composition pharmaceutique comprenant un système d'administration nanostructuré selon l'une quelconque des revendications 1 à 5 ainsi que des excipients et additifs appropriés pour une utilisation dans le traitement d'un sujet souffrant d'un adénocarcinome, dans laquelle l'adénocarcinome est un cancer choisi dans le groupe consistant en le cancer gastro-intestinal, le cancer du poumon, le cancer de la prostate, le cancer du côlon et le cancer du sein, et comprend des cellules d'adénocarcinome avec une altération d'expression génique dans un ou plusieurs gènes *SLCO1.*

7. - Composition pharmaceutique pour l'utilisation selon la revendication 6, spécifiquement adaptée pour l'inhalation.

8. - Procédé pour le transport ciblé dans un tissu d'adénocarcinome et/ou une cellule d'adénocarcinome, dans lequel l'adénocarcinome est un cancer choisi dans le groupe consistant en le cancer gastro-intestinal, le cancer du poumon, le cancer de la prostate, le cancer du côlon et le cancer du sein, et comprend des cellules d'adénocarcinome avec une altération d'expression génique dans un ou plusieurs gènes *SLCO1,* comprenant la mise en contact du tissu d'adénocarcinome et/ou de la cellule d'adénocarcinome *in vitro* avec un système d'administration nanostructuré selon l'une quelconque des revendications 1 à 5 ou une composition pharmaceutique selon la revendication 6 ou 7.

9. - Procédé selon la revendication 8, dans lequel le tissu d'adénocarcinome est un tissu de prostate.

10. - Utilisation in vitro d'un système d'administration nanostructuré selon l'une quelconque des revendications 1 à 5 ou utilisation d'une composition pharmaceutique selon la revendication 6 ou 7, dans laquelle l'accumulation du système d'administration nanostructuré et/ou de ses composants est détectable *in vitro* dans le tissu d'adénocarcinome et/ou la cellule d'adénocarcinome, l'adénocarcinome étant un cancer choisi dans le groupe consistant en le cancer gastro-intestinal, le cancer du poumon, le cancer de la prostate, le cancer du côlon et le cancer du sein, et comprenant des cellules d'adénocarcinome avec une altération d'expression génique dans un ou plusieurs gènes *SLCO1,* au moyen des propriétés de fluorescence dudit au moins un colorant polyméthine.
